# EUROPEAN PATENT APPLICATION

(11) **EP 3 854 791 A1**
(43) Date of publication of application: **28.07.2021**
(21) Application number: 19861455.4
(22) Date of filing: 09.09.2019
(51) Int. Cl.: C07D 403/12, C07D 403/04, C07D 401/12, C07D 237/20, A01N 43/58, A01P 13/00

(54) **PREPARATION OF PYRIDAZINYL AMINE COMPOUND AND APPLICATION THEREOF**

(30) Priority: 20.09.2018 CN 201811103367
(71) Applicant: Synwill Co., Ltd., Taizhou, Zhejiang 318000 (CN)
(72) Inventor: ZHOU, Yinping, Nanjing, Jiangsu 210047 (CN)
(74) Representative: Zenz Patentanwälte Partnerschaft mbB
(86) International application number: PCT/CN2019/104960
(87) International publication number: WO 2020/057391

(57) **Abstract**

Provided is a pyridazinyl amine compound with broad-spectrum herbicidal activity and having a structure represented by general formula (I). The definition of each substituent in the formula is described in the description. The compound has broad-spectrum herbicidal activity, has a good control effect against *Echinochloa crus-galli, Eleusine indica,* and *Digitaria sanguinalis,* and can be used for wheat, soybean, and rice fields, orchards, and non-arable land to control various malignant weeds.

## Description

### Cross-Reference to Related Applications

The present disclosure claims priority to Chinese Patent Application No. CN201811103367.0, filed with the Chinese Patent Office on September 20, 2018, entitled "Preparation of Pyridazinyl Amine Compounds and Use Thereof", which is incorporated herein by reference in its entirety.

### Technical Field

The present disclosure pertains to the field of agricultural herbicides, and relates to a new type of pyridazinyl amine compounds and compositions, use thereof, and methods of using the same.

### Background Art

Weeds in farmland are an important factor affecting the production of crops. There are 43 million hectares of farmland invaded by weeds in China, whereby grain yield is reduced by 17.5 million tons every year. Chemical control of weeds is the most important means in the control of weeds in farmland, which has the advantages of saving labor, saving time, having high efficiency, and acting quickly and is an important measure to increase labor productivity and develop agriculture with high efficiency and high quality. Pyridazinyl amine compounds are generally used in medicine for anti-cancer and anti-inflammatory purposes. The pyridazinyl amine compounds of the present disclosure have excellent herbicidal activity, but the use of such structures as herbicides has not been reported in the literature. These compounds can be used as herbicides in agriculture.

### Summary

In order to meet the requirements in agriculture, the present disclosure provides a novel pyridazinyl amine compound, a method of using the same for controlling or eliminating weeds, and use of compositions containing these compounds in agriculture.

The technical solution of the present disclosure is implemented as follows.

The present disclosure provides a pyridazinyl amine compound as represented by general formula I or an N-oxide or agriculturally acceptable salt of the compound of general formula (I), 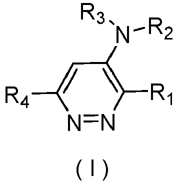 wherein
R₁ is selected from hydrogen, halogen, cyano, and phenoxy or heteroaryloxy unsubstituted or substituted with one to four groups independently selected from the group consisting of: halogen, nitro, cyano, C₁-C₁₂ alkyl, halogenated C₁-C₁₂ alkyl, C₁-C₁₂ alkoxy, halogenated C₁-C₁₂ alkoxy, C₃-C₁₂ cycloalkyl, C₁-C₁₂ alkylcarbonyl or C₁-C₁₂ alkoxycarbonyl;
R₂ is selected from hydrogen, C₁-C₁₂ alkyl, halogenated C₁-C₁₂ alkyl, C₁-C₁₂ alkoxy, halogenated C₁-C₁₂ alkoxy, C₃-C₆ cycloalkyl, cyano C₁-C₁₂ alkyl, C₁-C₁₂ alkylamino, halogenated C₁-C₁₂ alkylamino, C₂-C₆ dialkylamino, C₂-C₁₂ alkenyl, halogenated C₂-C₁₂ alkynyl, C₂-C₁₂ alkenyloxy, halogenated C₂-C₁₂ alkenyloxy, C₂-C₁₂ alkynyl, halogenated C₂-C₁₂ alkenyl, C₂-C₁₂ alkynyloxy, halogenated C₂-C₁₂ alkynyloxy, C₁-C₁₂ alkylsulfonyl, C₁-C₁₂ alkylcarbonyl, halogenated C₁-C₁₂ alkylcarbonyl, or halogenated C₁-C₁₂ alkylsulfonyl;
R₃ is selected from phenyl, benzyl or heteroaryl unsubstituted or substituted with one to four groups independently selected from the group consisting of: halogen, hydroxyl, nitro, cyano, CHO, COOH, COONa, C₁-C₁₂ alkyl, halogenated C₁-C₁₂ alkyl, C₁-C₁₂ alkoxy, halogenated C₁-C₁₂ alkoxy, C₃-C₆ cycloalkyl, cyano C₁-C₁₂ alkyl, C₁-C₁₂ alkylamino, halogenated C₁-C₁₂ alkylamino, C₂-C₆ dialkylamino, C₂-C₁₂ alkenyl, halogenated C₂-C₁₂ alkynyl, C₂-C₁₂ alkenyloxy, halogenated C₂-C₁₂ alkenyloxy, C₂-C₁₂ alkynyl, halogenated C₂-C₁₂ alkenyl, C₂-C₁₂ alkynyloxy, halogenated C₂-C₁₂ alkynyloxy, C₁-C₁₂ alkylthio, halogenated C₁-C₁₂ alkylthio, C₁-C₁₂ alkylsulfonyl, C₁-C₁₂ alkylcarbonyl, halogenated C₁-C₁₂ alkylcarbonyl or halogenated C₁-C₁₂ alkylsulfonyl;
R₂NR₃ may form a five-membered or six-membered ring unsubstituted or substituted with one to four groups (substituents) independently selected from the group consisting of: halogen, hydroxyl, nitro, cyano, CHO, COOH, COONa, C₁-C₁₂ alkyl, halogenated C₁-C₁₂ alkyl, C₃-C₁₂ cycloalkyl, C₁-C₁₂ alkoxy, halogenated C₁-C₁₂ alkoxy, C₁-C₁₂ alkylthio, C₁-C₁₂ alkylcarbonyl or C₁-C₁₂ alkoxycarbonyl;
R₄ is selected from halogen, cyano, CHO, COOH, COONa, C₁-C₁₂ alkylsulfonyl, halogenated C₁-C₁₂ alkylsulfonyl, or arylsulfonyl unsubstituted or substituted with one to four substituents independently selected from the group consisting of: halogen, hydroxyl, nitro, cyano, CHO, COOH, COONa, C₁-C₁₂ alkyl, halogenated C₁-C₁₂ alkyl, C₃-C₁₂ cycloalkyl, C₁-C₁₂ alkoxy, halogenated C₁-C₁₂ alkoxy, C₁-C₁₂ alkylthio, C₁-C₁₂ alkylcarbonyl or C₁-C₁₂ alkoxycarbonyl.

The present disclosure provides a preferable compound or an N-oxide or agriculturally acceptable salt of the compound of general formula (I), wherein in the general formula (I),
R₁ is selected from hydrogen, Cl, Br, F, I, cyano, and phenoxy or heteroaryloxy unsubstituted or substituted with one to four groups independently selected from the group consisting of: halogen, nitro, cyano, C₁-C₆ alkyl, halogenated C₁-C₆ alkyl, C₁-C₆ alkoxy, halogenated C₁-C₆ alkoxy, C₃-C₆ cycloalkyl, C₁-C₆ alkylcarbonyl or C₁-C₆ alkoxycarbonyl;
R₂ is selected from hydrogen, C₁-C₆ alkyl, halogenated C₁-C₆ alkyl, C₁-C₆ alkoxy, halogenated C₁-C₆ alkoxy, C₃-C₆ cycloalkyl, cyano C₁-C₆ alkyl, C₁-C₆ alkylamino, halogenated C₁-C₆ alkylamino, C₂-C₆ dialkylamino, C₂-C₆ alkenyl, halogenated C₂-C₆ alkenyl, C₂-C₆ alkenyloxy, halogenated C₂-C₆ alkenyloxy, C₂-C₆ alkynyl, halogenated C₂-C₆ alkynyl, C₂-C₆ alkynyloxy, halogenated C₂-C₆ alkynyloxy, C₁-C₆ alkylsulfonyl, C₁-C₆ alkylcarbonyl, halogenated C₁-C₆ alkylcarbonyl, or halogenated C₁-C₆ alkylsulfonyl;
R₃ is selected from phenyl, benzyl or heteroaryl unsubstituted or substituted with one to four groups independently selected from the group consisting of: Cl, Br, F, I, hydroxyl, nitro, cyano, CHO, COOH, COONa, C₁-C₆ alkyl, halogenated C₁-C₆ alkyl, C₁-C₆ alkoxy, halogenated C₁-C₆ alkoxy, C₃-C₆ cycloalkyl, cyano C₁-C₆ alkyl, C₁-C₆ alkylamino, halogenated C₁-C₆ alkylamino, C₂-C₆ dialkylamino, C₂-C₆ alkenyl, halogenated C₂-C₆ alkynyl, C₂-C₆ alkenyloxy, halogenated C₂-C₆ alkenyloxy, C₂-C₆ alkynyl, halogenated C₂-C₆ alkenyl, C₂-C₆ alkynyloxy, halogenated C₂-C₆ alkynyloxy, C₁-C₆ alkylthio, halogenated C₁-C₆ alkylthio, C₁-C₆ alkylsulfonyl, C₁-C₆ alkylcarbonyl, halogenated C₁-C₆ alkylcarbonyl or halogenated C₁-C₆ alkylsulfonyl;
R₂NR₃ may form a five-membered or six-membered ring unsubstituted or substituted with one to four substituents independently selected from the group consisting of: Cl, Br, F, I, hydroxyl, nitro, cyano, CHO, COOH, COONa, C₁-C₆ alkyl, halogenated C₁-C₆ alkyl, C₃-C₆ cycloalkyl, C₁-C₆ alkoxy, halogenated C₁-C₆ alkoxy, C₁-C₆ alkylthio, C₁-C₆ alkylcarbonyl or C₁-C₆ alkoxycarbonyl;
R₄ is selected from Cl, Br, F, I, cyano, CHO, COOH, COONa, C₁-C₆ alkylsulfonyl, or halogenated C₁-C₆ alkylsulfonyl.

The present disclosure provides a further preferable compound, wherein in the general formula (I),
R₁ is selected from H, Cl, Br, F, I, CN, CH₃, CH₂CH₃, tert-butyl, cyclopropyl, CF₃, CH₂CF₃, OCH₃, OCF₃, OCH₂CF₃, SO₂CH₃, CO₂CH₃, and phenoxy unsubstituted or substituted with a substituent(s) selected from one to three of Cl, Br, F, I, CN, NO₂, CHO, COOH, COONa, CH₃, CH₂CH₃, tert-butyl, cyclopropyl, CHF₂, CF(CF₃)₂, CF₃, CH₂CF₃, OCH₃, OCHF₂, OCF₃, OCH₂CF₃ or SO₂CH₃;
R₂ is selected from H, methyl, ethyl, propyl, isopropyl, tert-butyl, n-butyl, methoxy, cyclopropyl, cyanoethyl, methylamino, methanesulfonyl, allyl, propargyl, methanesulfonyl, p-toluenesulfonyl, ethylcarbonyl, or formyl;
R₃ is selected from phenyl, benzyl or heteroaryl unsubstituted or substituted with one to three groups independently selected from the group consisting of: OH, Cl, Br, F, I, CN, NO₂, CHO, COOH, COONa, CH₃, CH₂CH₃, tert-butyl, cyclopropyl, CHF₂, CF₃, CF(CF₃)₂, CH₂CF₃, OCH₃, OCHF₂, OCF₃, OCH₂CF₃ or SO₂CH₃;
R₂NR₃ may form a five-membered or six-membered ring unsubstituted or substituted with one to three substituents independently selected from the group consisting of: OH, Cl, Br, F, I, CN, NO₂, CH₃, CH₂CH₃, tert-butyl, cyclopropyl, CHF₂, CF(CF₃)₂, CF₃, CH₂CF₃, OCH₃, OCHF₂, OCF₃, OCH₂CF₃ or SO₂CH₃;
R4 is selected from Cl, Br, F, I, CN, SO₂CH₃, or SO₂CH₂CH₃;
or an N-oxide or a salt of the compound of general formula (I),
wherein the salt is formed by the compound of general formal (I) and hydrochloric acid, sulfuric acid, phosphoric acid, formic acid, acetic acid, oxalic acid, trifluoroacetic acid, methanesulfonic acid, p-toluenesulfonic acid, malic acid, citric acid, lithium, calcium, sodium, or potassium.

The present disclosure provides a still further preferable compound, wherein in the general formula (I),
R₁ is selected from Cl, Br, F, I, 2-methylphenol, 2-isopropylphenol, 2-cyclopropylphenol, 2-methyl-6-cyclopropylphenol, or 2-iodophenol;
R₂ is selected from H, CH₃, CH₂CH₃, cyclopropyl, methoxy, or methylamino;
R₃ is selected from phenyl, pyrimidinyl, pyrazolyl, pyridinyl or pyridazinyl unsubstituted or substituted with one to three groups independently selected from the group consisting of: OH, Cl, Br, F, I, CN, NO₂, CHO, COOH, COONa, CH₃, CH₂CH₃, tert-butyl, cyclopropyl, CHF₂, CF₃, CH₂CF₃, OCH₃, OCHF₂, OCF₃, CF(CF₃)₂, OCH₂CF₃ or SO₂CH₃;
R₂NR₃ may form morpholine, piperazine, piperidine, a pyrrole ring, a pyrazole ring, a triazole ring, a pyrimidine ring, or 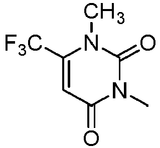 or the like;
R₄ is selected from Cl, Br, F, I, or CN;
or a salt formed by the compound of general formula (I) and hydrochloric acid, sulfuric acid, phosphoric acid, lithium, calcium, sodium, or potassium.

The present disclosure provides a still further preferable compound, wherein in the general formula (I),
R₁ is selected from Cl, Br, F, or I;
R₂ is selected from H, CH₃, or CH₂CH₃;
R₃ is selected from phenyl, pyridyl or pyridazinyl unsubstituted or substituted with one to three groups independently selected from the group consisting of: OH, Cl, Br, F, I, CN, NO₂, CHO, COOH, COONa, CH₃, CH₂CH₃, tert-butyl, CHF₂, CF₃, CH₂CF₃, CF(CF₃)₂, OCH₃, OCHF₂, OCF₃, OCH₂CF₃ or SO₂CH₃;
R₂NR₃ may form a ring 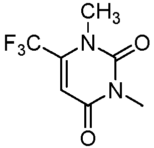 or the like;
R₄ is selected from Cl, Br, F, I, or CN;
or a salt formed by the compound of general formal (I) and hydrochloric acid, sulfuric acid, phosphoric acid, lithium, calcium, sodium, or potassium.

In the compounds of the present disclosure, stereoisomers (with different configurations denoted by R and S, respectively) can be formed due to the chiral carbon or nitrogen being connected to different groups or substituents. The present disclosure includes R-isomers and S-isomers and mixtures thereof in any ratio.

In the definitions of the compound of general formula (I) given above, general definitions of the terms used are summarized as follows:
The term "unsubstituted" means that all substituents are hydrogen.

The term "halogen" refers to fluorine, chlorine, bromine, or iodine.

The term "alkyl" refers to a linear (or straight), branched, or cyclized alkyl group, such as methyl, ethyl, propyl, isopropyl, tert-butyl or cyclopropyl.

The term "halogenated alkyl" refers to linear or branched halogenated alkyl groups in which some or all of hydrogen atoms may be substituted with halogen atoms, for example, halogenated alkyl groups such as chloromethyl, dichloromethyl, trichloromethyl, fluoromethyl, difluoromethyl or trifluoromethyl.

The term "alkoxy" refers to a linear, branched, or cyclized alkoxy group, such as methoxy, ethoxy, propoxy, isopropoxy, tert-butoxy or cyclopropoxy.

The term "halogenated alkoxy" refers to linear or branched halogenated alkoxy groups in which some or all of hydrogen atoms may be substituted with halogen atoms, for example, halogenated alkoxy groups such as chloromethoxy, dichloromethoxy, trichloromethoxy, fluoromethoxy, difluoromethoxy or trifluoromethoxy.

The term "alkylthio" refers to a linear or branched alkyl group connected to a structure via a sulfur atom bond.

The term "halogenated alkylthio" refers to linear or branched alkylthio groups in which some or all of hydrogen atoms may be substituted with halogen atoms, such as chloromethylthio, dichloromethylthio, trichloromethylthio, fluoromethylthio, difluoromethylthio, trifluoromethylthio, chlorofluoromethylthio, or the like.

The term "alkenyl" refers to a linear or branched chain having double bonds in any position, such as vinyl or allyl.

The term "alkynyl" refers to a linear or branched chain having triple bonds in any position, such as ethynyl or propargyl.

An aryl group and an aryl moiety in an aralkyl group includes phenyl, naphthyl or the like.

The term "heteroaryl" mentioned in the present disclosure is a five-membered ring, six-membered ring, or fused heterocyclic compound containing one or more N, O, or S heteroatoms, such as morpholine, piperazine, piperidine, pyrrole, pyridine, furan, pyrimidine, pyrazine, pyridazine, triazine, quinoline, isoquinoline, indole, purine, benzopyrone, or the like.

The present disclosure provides a salt obtained by addition of a pyridazinyl amine represented by the following general formula (II) to an acid or base: 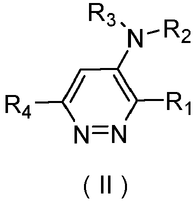 where R₁, R₂, R₃, and R₄ have the same meanings as those defined in the general formula (I).

Acids that can form salts with the pyridazinyl amine represented by the general formula (II) according to the present disclosure include: carboxylic acids, such as acetic acid, propionic acid, butyric acid, oxalic acid, or trifluoroacetic acid; sulfonic acids, such as methanesulfonic acid, p-toluenesulfonic acid, or dodecylbenzenesulfonic acid; and mineral acids, such as hydrochloric acid, sulfuric acid, nitric acid, or carbonic acid.

Bases and metals that can form salts with the pyridazinyl amine represented by the general formula (II) according to the present disclosure include: metals, such as sodium, potassium, calcium, magnesium, or the like; organic bases, such as sodium methoxide, sodium ethoxide, sodium tert-butoxide, potassium methoxide, potassium ethoxide, sodium formate, butyl lithium, sodium acetate, or the like; and inorganic bases, such as sodium hydroxide, potassium hydroxide, sodium carbonate, potassium carbonate, sodium hydride, sodium amide, or the like.

Table 1 and Table 2 respectively list some specific substituents as R₁ and R₄ in the general formula I, but they are not limited to these substituents. 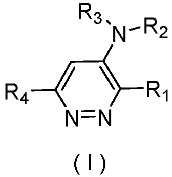 R₁ represents some specific substituents

**Table 1**

| R₁ | R₁ | R₁ | R₁ | R₁ |
|---|---|---|---|---|
| H | F | Cl | Br | I |
| CHO | CN | COOH | 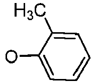 | 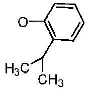 |
| 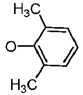 | 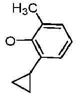 | 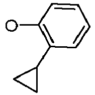 | 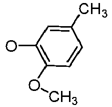 | 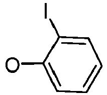 |

R₄ represents some specific substituents

**Table 2**

| R₄ | R₄ | R₄ | R₄ | R₄ |
|---|---|---|---|---|
| H | F | Cl | Br | I |
| CHO | CN | COOH | SO₂CH₃ | SO₂CH₂CH₃ |

**Table 3 Substituent represented by R₂NR₃ when a cyclic structure is formed**

| R₂NR₃ | R₂NR₃ | R₂NR₃ | R₂NR₃ | R₂NR₃ |
|---|---|---|---|---|
| 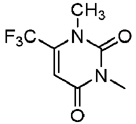 | 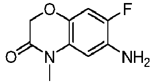 | 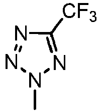 | 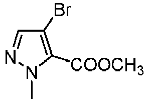 | 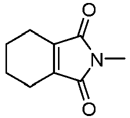 |

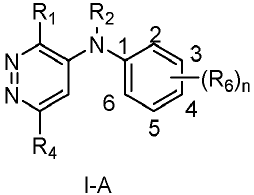

**Table 4**

| (R₆)ₙ | (R₆)ₙ | (R₆)ₙ |
|---|---|---|
| 2-F | 2-Br | 2-CH₃-4-Cl-6-CON(CH₃)₂ |
| 2,4-2F | 2,6-2Br-4-NO₂ | 2-CH₃-4-Cl-6-CONHCH(CH₃)₂ |
| 2,6-2F | 2,6-2Br-4-OCF₃ | 2-CH₃-4-Cl-6-CONHC(CH₃)₃ |
| 2-F-5-CH₃ | 3-Br-4-CN | 2-CH₃-4-Br-6-CO₂CH₃ |
| 2-F-5-NO₂ | 4-Br | 2-CH₃-4-Br-6-CONH₂ |
| 2-F-4-CN | 2-1 | 2-CH₃-4-Br-6-CON(CH₃)₂ |
| 2,3,4-3F | 4-1 | 2-CH₃-4-NO₂-6-F |
| 2-F-4-NO₂ | 2-NO₂ | 2-CH₃-4-NO₂-6-Cl |
| 2,6-2F-4-CF₃ | 2-NO₂-4-Cl | 2-CH₃-4-NO₂-6-Br |
| 2,5-2F-4-CO₂C₂CH₅ | 2-NO₂-4-CN | 2-CH₃-4-NO₂-6-CN |
| 2,6-2F-4-NO₂ | 2-NO₂-5-Cl | 2-CH₃-4-NO₂-6-CF₃ |
| 2,6-2F-4-OCF₃ | 2,6-2NO₂-4-Cl | 2-CH₃-4-NO₂-6-OCH₃ |
| 2-F-4-NO₂-6-Cl | 2,6-2NO₂-4-SOCH₃ | 2-CH₃-4-NO₂-6-SO₂CH₃ |
| 2-F-4-CN-5-CO₂CH₃ | 2,6-2NO₂-4-SO₂CH | 2-CH₃-4-NO₂-6-CO₂H |
| 2-F-4-CN-5-NO₂ | 2-NO₂-4,6-2Cl | 2-CH₃-4-NO₂-6-CO₂CH₃ |
| 2-F-4-CN-6-OCH₃ | 2-NO₂-4-Cl-6-F | 2-CH₃-4,6-2CN |
| 2-F-4-NO₂-6-Cl | 2-NO₂-4-CN-6-Cl | 2-CH₃-4-CN-6-NO₂ |
| 2-F-4-NO₂-6-Br | 2-NO₂-3-C₂H₅-6-CO₂H₃ | 2-CH₃-4-CN-6-CF₃ |
| 2-F-4-CN-6-Cl | 2-NO₂-4-CO₂H₃-6-SCH₃ | 4-CH₃ |

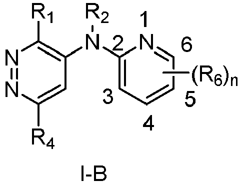

**Table 5**

| (R₆)ₙ | (R₆)ₙ | (R₆)ₙ | (R₆)ₙ |
|---|---|---|---|
| 3-1 | 5-CHO | 4-I-5-CH₃ | 3,5-2F-4-CF₃ |
| 4-Cl | 3-NH₂ | 4-I-5-CF₃ | 3,6-2F-4-CF₃ |
| 4-Br | 4-NH₂ | 5-I-6-C₂H₅ | 3-F-5-CN-6-Cl |
| 4-1 | 5-NH₂ | 3-NO₂-4-Cl | 3-F-5-CO₂CH₃-6-Cl |
| 5-Cl | 6-NH₂ | 3-NO₂-4-CH₃ | 3-F-5-CONH₂-6-Cl |
| 5-Br | 3-CONH₂ | 3-NO₂-5-Cl | 3-F-5-CONHCH(CH₃)₂-6-Cl |
| 5-1 | 4-CONH₂ | 3-NO₂-5-Br | 3,5-2Cl-4-CH₃ |
| 6-Cl | 5-CONH₂ | 3-NO₂-5-I | 3,5-2Cl-6-CH₃ |
| 6-Br | 3-F-5-Cl | 3-NO₂-6-Cl | 3,5-2Cl-4,6-2CH₃ |
| 3-NO₂ | 3-F-5-CN | 3-NO₂-6-CH₃ | 3,5-2Cl-6-OCH₂CO₂CH₃ |
| 4-NO₂ | 3-F-4-CF₃ | 3-NO₂-6-OCH₃ | 3,6-2Cl-5-CF₃ |
| 5-NO₂ | 3-F-5-CF₃ | 4-NO₂-6-CH₃ | 3,5,6-3Cl |
| 6-NO₂ | 3-F-4-CHO | 5-NO₂-6-Cl | 3-Br-5-CF₃-6-Cl |
| 3-CN | 4,6-2F | 5-NO₂-6-CH₃ | 3,5-2Br-4-CH₃ |
| 4-CN | 5-F-6-CH₃ | 5-NO₂-6-NHCOCH₃ | 3,5-2Br-6-CH₃ |
| 5-CN | 3,5-2Cl | 3-CN-6-CH₃ | 3-Br-5,6-2CH₃ |
| 6-CN | 4,6-2Cl | 3-CN-4-OCH₃ | 3-NO₂-4-CH₃-5-Br |
| 3-CH₃ | 3,5-2Br | 3-CH₃-5-CN | 3,5-2CN-6-Cl |
| 4-CH₃ | 3-Cl-5-Br | 4-CH₃-5-Br | 3-CN-5-F-6-Cl |

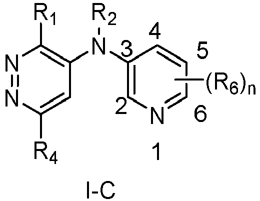

**Table 6**

| (R₆)ₙ | (R₆)ₙ | (R₆)ₙ | (R₆)ₙ |
|---|---|---|---|
| H | 2-CH₃ | 2-Cl-5-CH₃ | 5-CO₂H-6-Cl |
| 2-F | 4-CH₃ | 2-Cl-5-CF₃ | 5-CO₂CH₃-6-Cl |
| 2-Br | 4-CF₃ | 2-Cl-4-CHO | 5-CONH₂-6-Cl |
| 2-Cl | 5-CF₃ | 2-Cl-5-SO₂NH₂ | 2,5,6-3F |
| 2-1 | 6-CF₃ | 5-Cl-6-CN | 2,6-2F-5-Cl |

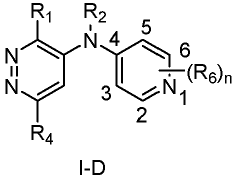

**Table 7**

| (R₆)ₙ | (R₆)ₙ | (R₆)ₙ | (R₆)ₙ |
|---|---|---|---|
| H | 2-CF₃ | 3,5-2Cl | 2,6-2NO₂ |
| 2-F | 3-CF₃ | 3,5-2Br | 3,5-2CH₃ |
| 3-F | 5-CF₃ | 3,5-21 | 2,6-2CH₃ |
| 2-Cl | 2-OCH₃ | 2-F-5-CH₃ | 3-CH₃-2-Cl |
| 2-Br | 3-OCH₃ | 2-F-3-CHO | 3,6-2Cl-5-CF₃ |

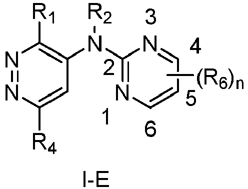

**Table 8**

| (R₆)ₙ | (R₆)ₙ | (R₆)ₙ | (R₆)ₙ |
|---|---|---|---|
| H | 5-CO₂C₂H₅ | 4-Cl-5-NO₂ | 4-CF₃-5-CO₂C₂H₅ |
| 4-F | 5-CO₂C(CH₃)₃ | 4-Cl-5-CN | 4-CF₃-5-CONH₂ |
| 4-Cl | 5-CHO | 4-Cl-5-CH₃ | 4-OCH₃-5-Br |
| 4-CN | 5-NH₂ | 4-Cl-6-CH₃ | 4,6-2OCH₃ |
| 4-NO₂ | 5-CONH₂ | 4-Cl-6-CH(CH₃)₂ | 4-CO₂CH₃-6-Cl |
| 4-CH₃ | 5-CONHCH₃ | 4-Cl-5-CF₃ | 4-CO₂C₂H₅-6-Cl |
| 4-CF₃ | 5-CONHC₂H₅ | 4-Cl-5-OCH₃ | 4-CO₂CH₃-6-CH₃ |
| 4-OCH₃ | 5-CONHCH(CH₃)₂ | 4-Cl-6-OCH₃ | 4-CO₂C₂H₅-6-CH₃ |

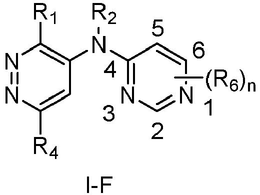

**Table 9**

| (R₆)ₙ | (R₆)ₙ | (R₆)ₙ | (R₆)ₙ |
|---|---|---|---|
| H | 2,5-2Cl | 2-CH₃-6-Cl | 2-SCH₃-5-CONH₂ |
| 2-F | 2,6-2Cl | 5-CH₃-6-Cl | 2-SO₂CH₃-6-Cl |
| 2-Cl | 2-Cl-5-Br | 2-CH₃-5-CN | 2-SO₂CH₃-5-Br |
| 2-OC₂H₅ | 2-Cl-5-I | 2-CH₃-5-C(CH₃)₃ | 2-SO₂CH₃-6-OCH₃ |
| 2-SCH₃ | 2-Cl-5-NO₂ | 2-CF₃-5-CO₂C₂H₅ | 5-CHO-6-Cl |
| 2-SOCH₃ | 2-Cl-5-CN | 2-OCH₃-6-Cl | 5-CHO-6-NH₂ |
| 2-SO₂CH₃ | 2-Cl-5-CH₃ | 2-SCH₃-6-Cl | 5-CO₂H-6-Cl |
| 2-NHCH₃ | 2-Cl-6-CH₃ | 2-SCH₃-5-Br | 2-NH₂-6-CH(CH₃)₂ |

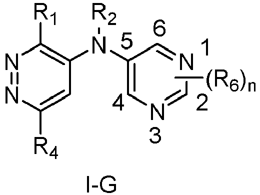

**Table 10**

| (R₆)ₙ | (R₆)ₙ | (R₆)ₙ | (R₆)ₙ |
|---|---|---|---|
| H | 4-Cl | 2,4-2Br | 2-SCH₃-4-CO₂CH₃ |
| 2-F | 4-Br | 4,6-2Br | 2-SCH₃-4-CO₂H |
| 2-Cl | 2,4-2F | 2-OCH₃-4-Cl | 2-SCH₃-4-CONH₂ |
| 2-Br | 4,6-2F | 2-OCH₃-4-Br | 2-SCH₃-4-CSNH₂ |
| 2-1 | 2,4,6-3F | 2,4-2OCH₃ | 2-SO₂CH₃-4-Cl |
| 2-CN | 2,4-2Cl | 4,6-2OCH₃ | 2-CH₃-4,6-2Cl |

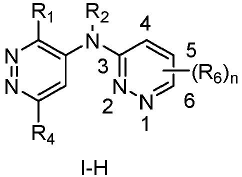

**Table 11**

| (R₆)ₙ | (R₆)ₙ | (R₆)ₙ | (R₆)ₙ |
|---|---|---|---|
| 6-Cl | 6-CO₂CH₃ | 6-CON(CH₃)₂ | 4-Cl-6-CO₂CH₃ |
| 6-Br | 6-CO₂C₂H₅ | 6-CON(C₂H₅)₂ | 4-Cl-6-CONHCH₃ |
| H | 6-CO₂C(CH₃)₃ | 6-CSNH₂ | 5,6-2Cl |
| 6-CN | 6-CO₂H | 6-CSNHCH₃ | 4-Br-6-CN |
| 6-CH₃ | 6-NH₂ | 4-F-6-CN | 4-Br-6-CO₂CH₃ |
| 6-OCH₃ | 6-N(CH₃)₂ | 4-F-6-CO₂CH₃ | 4-Br-6-CONHCH₃ |
| 6-SCH₃ | 6-CONH₂ | 4-F-6-CONHCH₃ | 4-CH₃-6-CN |
| 6-SOCH₃ | 6-CONHCH₃ | 3-Cl-5-NHCOCH₃ | 4-CH₃-6-CO₂CH₃ |

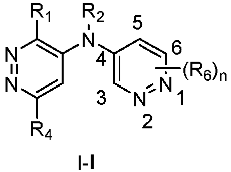

**Table 12**

| (R₆)ₙ | (R₆)ₙ | (R₆)ₙ | (R₆)ₙ |
|---|---|---|---|
| H | 5,6-2Cl | 5-Cl-6-OCH₃ | 3-Cl-5-NHCOCH₃ |
| 3-Cl | 3-Cl-6-OCH₃ | 5-Cl-6-SCH₃ | 3-Cl-5-NHCO₂CH₃ |
| 6-Cl | 3-Cl-6-SCH₃ | 5-Cl-6-SOCH₃ | 5,6-2OCH₃ |
| 3,6-2Cl | 5-Cl-6-N(CH₃)₂ | 5-Cl-6-SO₂CH₃ | 5,6-2SCH₃ |
| 3,6-2F | 3,6-2Br | 3,6-21 | 3-F-6-Cl |
| 3-F-6-Br | 3-F-6-I | 3-Cl-6-F | 3-Cl-6-Br |
| 3-Cl-6-I | 3-Br-6-F | 3-Br-6-Cl | 3-Br-6-I |
| 3-I-6-F | 3-I-6-Cl | 3-I-6-Br | 3-SO₂CH₃-6-Cl |
| 3-SO₂C₂H₅-6-Cl | 3-CN-6-Cl | 3-Cl-6-CF₃ | 3-CN-6-CF₃ |

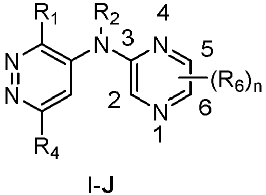

**Table 13**

| (R₆)ₙ | (R₆)ₙ | (R₆)ₙ | (R₆)ₙ |
|---|---|---|---|
| H | 6-CO₂CH₃ | 6-CONHCH₃ | 4-CH₃-6-CN |
| 6-Cl | 6-CO₂C₂H₅ | 6-CONHC₂H₅ | 4-F-6-CO₂CH₃ |
| 6-Br | 6-CO₂C(CH₃)₃ | 6-CON(CH₃)₂ | 4-Cl-6-CO₂CH₃ |
| 6-CN | 6-CO₂H | 6-CSNH₂ | 4-Br-6-CO₂CH₃ |
| 6-CH₃ | 6-CO₂Na | 6-CSNHCH₃ | 4-CH₃-6-CO₂CH₃ |
| 6-OCH₃ | 6-CO₂NH₄ | 5,6-2Cl | 4-F-6-CONHCH₃ |
| 6-SCH₃ | 6-NH₂ | 4-F-6-CN | 4-Cl-6-CONHCH₃ |
| 6-SOCH₃ | 6-N(CH₃)₂ | 4-Cl-6-CN | 4-Br-6-CONHCH₃ |
| 6-SO₂CH₃ | 6-CONH₂ | 4-Br-6-CN | 4-CH₃-6-CONHCH₃ |

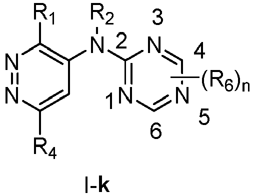

**Table 14**

| (R₆)ₙ | (R₆)ₙ | (R₆)ₙ | (R₆)ₙ |
|---|---|---|---|
| 4-Cl | 4-OCH₃-6-NHCH₃ | 4-N(CH₃)₂ | 4-NH-i-Pr-6-NHCOCH₃ |
| 4-CN | 4,6-2SCH₃ | 4-NH₂-6-Cl | 4-NH-i-Pr-6-NH₂ |
| 4-SCH₃ | 4-SCH₃-6-Cl | 4-NH₂-6-OCH₃ | 4-NH-i-Pr-6-NHCH₃ |
| 4-SOCH₃ | 4-SCH₃-6-NHCH₃ | 4-NH₂-6-SCH₃ | 4-NH-t-Bu-6-Cl |
| 4-SO₂CH₃ | 4-SOCH₃-6-Cl | 4-NH₂-6-SOCH₃ | 4-NH-t-Bu-6-CN |
| 4,6-2Cl | 4-SO₂CH₃-6-Cl | 4-NH₂-6-SO₂CH₃ | 4-NH-t-Bu-6-OCH₃ |
| 4-CN-6-Cl | 4-NHCOH₃-6-Cl | 4-NH-i-Pr-6-Cl | 4-NH-t-Bu-6-SCH₃ |
| 4,6-2CH₃ | 4-NHCOH₃-6-OCH₃ | 4-NH-i-Pr-6-CN | 4-NH-t-Bu-6-NH₂ |
| 4,6-2OCH₃ | 4-NHCOH₃-6-SCH₃ | 4-NH-i-Pr-6-OCH₃ | 4-NH-t-Bu-6-NHCH₃ |
| 4-OCH₃-6-Cl | 4-NHCOH₃-6-SO₂CH₃ | 4-NH-i-Pr-6-SCH₃ | 4-NH-t-Bu-6-NHCOH₃ |

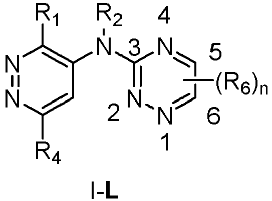

**Table 15**

| (R₆)ₙ | (R₆)ₙ | (R₆)ₙ | (R₆)ₙ |
|---|---|---|---|
| 5-F-6-CN | 5-Br-6-CO₂CH₃ | 6-CO₂CH₃ | 6-CONH(C₂H₅)₂ |
| 5-Cl-6-CN | 5-F-6-CONCH₃ | 6-CO₂H | 6-CSNH₂ |
| 5-Br-6-CN | 5-Cl-6-CONCH₃ | 6-CONH₂ | 6-CSNHCH₃ |
| 5-CH₃-6-CN | 5-F-Br-CONCH₃ | 6-CONHCH₃ | 6-CSNHC₂H₅ |
| 5-F-6-CO₂CH₃ | 5-CH₃-6-CO₂CH₃ | 6-CONHC₂H₅ | 6-CSN(CH₃)₂ |
| 5-Cl-6-CO₂CH₃ | 6-CN | 6-CONH(CH₃)₂ | 6-CSN(C₂H₅)₂ |

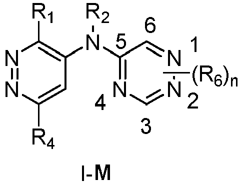

**Table 16**

| (R₆)ₙ | (R₆)ₙ | (R₆)ₙ | (R₆)ₙ |
|---|---|---|---|
| 3-CN | 3-CONHCH₃ | 3-CSN(CH₃)₂ | 3-CO₂CH₃-6-Cl |
| 3-CO₂CH₃ | 3-CONHC₂H₅ | 3-CSN(C₂H₅)₂ | 3-CO₂CH₃-6-Br |
| 3-CO₂C₂H₅ | 3-CON(CH₃)₂ | 3-CN-6-F | 3-CO₂CH₃-6-CH₃ |
| 3-CO₂H | 3-CON(C₂H₅)₂ | 3-CN-6-Cl | 3-CONHCH₃-6-F |
| 3-CO₂Na | 3-CSNH₂ | 3-CN-6-Br | 3-CONHCH₃-6-Cl |
| 3-CO₂NH₄ | 3-CSNHCH₃ | 3-CN-6-CH₃ | 3-CONHCH₃-6-Br |
| 3-CONH | 3-CSNHC₂H₅ | 3-CO₂CH₃-6-F | 3-CONHCH₃-6-CH₃ |

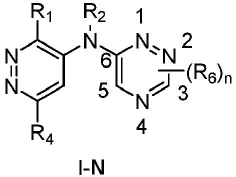

**Table 17**

| (R₆)ₙ | (R₆)ₙ | (R₆)ₙ | (R₆)ₙ |
|---|---|---|---|
| 3-CN | 3-CONH₂ | 3-CSNHC₂H₅ | 3-CO₂CH₃-5-Cl |
| 3-CO₂CH₃ | 3-CONHCH₃ | 3-CSN(CH₃)₂ | 3-CO₂CH₃-5-Br |
| 3-CO₂C₂H₅ | 3-CONHC₂H₅ | 3-CN-5-F | 3-CO₂CH₃-5-CH₃ |
| 3-CO₂C(CH₃)₃ | 3-CON(CH₃)₂ | 3-CN-5-Cl | 3-CONHCH₃-5-F |
| 3-CO₂H | 3-CON(C₂H₅)₂ | 3-CN-5-Br | 3-CONHCH₃-5-Cl |
| 3-CO₂Na | 3-CSNH₂ | 3-CN-5-CH₃ | 3-CONHCH₃-5-Br |
| 3-CO₂NH₄ | 3-CSNHCH₃ | 3-CO₂CH₃-5-F | 3-CONHCH₃-5-CH₃ |

Some of the compounds of the present disclosure may be illustrated by specific compounds listed in Table 18, but the present disclosure is not limited thereto.

**Table 18**

| | | | | |
|---|---|---|---|---|
| 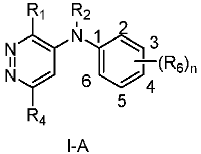 | | | | |

| No. | R₁ | R₂ | R₄ | (R₆)ₙ |
|---|---|---|---|---|
| I-A-1 | Cl | H | Cl | 2,3,4-3F |
| I-A-2 | Cl | H | Cl | 2-F-4-NO₂ |
| I-A-3 | Cl | H | Cl | 2-Cl |
| I-A-4 | Cl | H | Cl | 2-Cl-4-NO₂ |
| I-A-5 | Cl | H | Cl | 2-Cl-5-NO₂ |
| I-A-6 | Cl | H | Cl | 2-Cl-4-CF₃ |
| I-A-7 | Cl | H | Cl | 2-Cl-5-CF₃ |
| I-A-8 | Cl | H | Cl | 2,6-2Cl-4-NO₂ |
| I-A-9 | Cl | H | Cl | 2,6-2Br-4-NO₂ |
| I-A-10 | Cl | H | Cl | 2-NO₂-4-Cl |
| I-A-11 | Cl | H | Cl | 4-CN |
| I-A-12 | Cl | H | Cl | 2-CH₃-6-CO₂CH₃ |
| I-A-13 | Cl | H | Cl | 3-CF₃-4-CN |
| I-A-14 | Cl | H | Cl | 2-OCH₃-4-NO₂ |
| I-A-15 | Cl | H | Cl | 4-OCF₃ |
| I-A-16 | Cl | H | Cl | 2,6-2Cl-4-CF₃ |
| I-A-17 | Cl | H | Cl | 3,5,6-3Cl-2,4-2CN |
| I-A-18 | Cl | H | Cl | 2,6-2NO₂-4-CF₃ |
| I-A-19 | Cl | H | Cl | 2,6-2NO₂-3-Cl-4-CF₃ |
| I-A-20 | Cl | H | Cl | 2,6-2NO₂-3,4-2CH₃ |
| I-A-21 | Cl | H | Cl | 3,4-2Cl |
| I-A-22 | Cl | H | Cl | 2,6-2NO₂-4-t-Bu |
| I-A-23 | Cl | CH₂OCH₂CH₃ | Cl | 2-CH₃-6-CH₂CH₃ |
| I-A-24 | Cl | CH₂OCH₃ | Cl | 2,6-2CH₂CH₃ |
| I-A-25 | Cl | H | Cl | 2,4-2NO₂-3-N(CH₂CH₂CH₃)₂-6 -CF₃ |
| I-A-26 | Cl | H | Cl | 2-F-4-Cl-5-(cyclopentyloxy) |
| 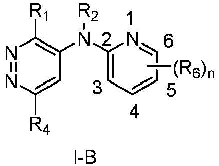 | | | | |
| No. | R₁ | R₂ | R₄ | (R₆)ₙ |
| I-B-1 | Cl | H | Cl | 3-Cl-5-CF₃ |
| I-B-2 | Cl | H | Cl | 3-Cl |
| I-B-3 | Cl | H | Cl | 5-Br |
| I-B-4 | Cl | H | Cl | 4-CH₃ |
| 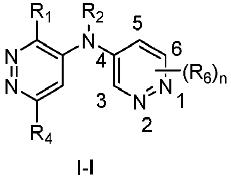 | | | | |
| No. | R₁ | R₂ | R₄ | (R₆)ₙ |
| I-I-1 | Cl | H | Cl | 3,6-2Cl |
| I-I-2 | Cl | H | Cl | 3,6-2Br |
| I-I-3 | Cl | H | Cl | 3,6-2F |
| I-I-4 | F | H | F | 3,6-2Br |
| I-I-5 | F | H | F | 3,6-2F |
| I-I-6 | Br | H | Br | 3,6-2Br |
| I-I-7 | Cl | CH₃ | Cl | 3,6-2Cl |
| I-I-8 | Cl | CH₂CH₃ | Cl | 3,6-2Cl |
| I-I-9 | Cl | CH(CH₃)₂ | Cl | 3,6-2Cl |
| I-I-10 | Cl | COCH₃ | Cl | 3,6-2Cl |
| I-I-11 | Cl | CH₂OCH₃ | Cl | 3,6-2Cl |
| I-I-12 | Cl | CH₂OCH₂CH₃ | Cl | 3,6-2Cl |
| I-I-13 | Cl | CH₂OCH₂CH₂CH₂CH₃ | Cl | 3,6-2Cl |
| I-I-13 | Cl | SO₂CH₃ | Cl | 3,6-2Cl |
| 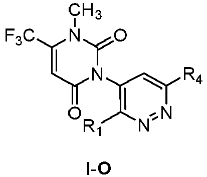 | | | | |
| No. | R₁ | | R₄ | |
| I-O-1 | H | | H | |
| I-0-2 | Cl | | Cl | |
| I-0-3 | F | | F | |
| I-0-4 | Br | | Br | |
| I-0-5 | 2-methylphenoxy | | Cl | |
| I-0-6 | 2-isopropylphenoxy | | Cl | |
| I-0-7 | 2-cyclopropyl | | Cl | |
| I-0-8 | 2-cyclopropyl-6-methylphenoxy | | Cl | |

### Synthesis Method

The compound of general formula (I) according to the present disclosure may be obtained from pyridazine compound II by means of the methods described in the literature, for example, in CN102924386B, CN104402828A, CN103058933A, and CN101099740.

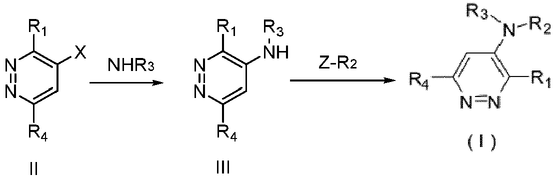

When R₂NR₃ represents a group 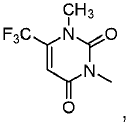 the compound may be prepared from compound A by means of literature, such as reference documents CN105753853A and CN103058933.

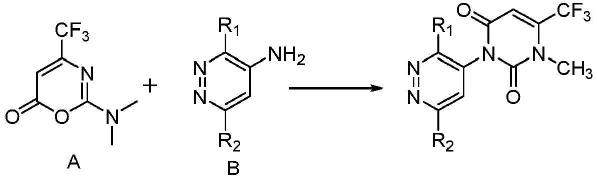

The reaction is carried out in a suitable solvent. The suitable solvent may be selected from benzene, toluene, xylene, acetone, tetrahydrofuran, acetonitrile, N,N-dimethylformamide, N-methylpyrrolidone, dichloromethane, chloroform, dichloroethane, ethyl acetate, or the like. The reaction may be carried out in the presence or absence of a base. When the reaction is carried out in the presence of a base, the reaction can be accelerated. The base may be selected from: alkali metal hydrides, such as sodium hydride, lithium hydride, sodium amide, or the like; alkali metal hydroxides, such as sodium hydroxide or potassium hydroxide; alkali metal carbonates, such as sodium carbonate or potassium carbonate; and organic bases, such as pyridine, 4-dimethylaminopyridine, triethylamine, N-methylpyrrole, diisopropylethylamine, or the like. The reaction may be carried out at a temperature between -10 °C and the boiling point temperature of the suitable solvent selected and used in the reaction, usually between 0 °C and 100 °C. The reaction is carried out for a time of 30 minutes to 20 hours, usually 1 to 10 hours.

All the intermediates mentioned herein are commercially available unless otherwise specified. X and Z are both leaving groups, and X and Z are selected from fluorine, chlorine, bromine, iodine, methanesulfonate, p-toluenesulfonate, or the like.

The compounds I and agriculturally acceptable salts thereof are suitable for use as herbicides in the forms of isomer mixtures and pure isomers. They are suitable for use as such or as appropriately formulated compositions. Herbicidal compositions comprising the compounds I are very effective in controlling plant growth in non-crop areas, especially at high application rates. They act against broad-leaved weeds and gramineous weeds in crops such as wheat, rice, corn, soybean and cotton, without inflicting any significant damage on the crops. This effect is mainly observed at low application rates.

Depending on the application method, the compounds I or compositions comprising them can additionally be used in many other crops to eliminate undesirable plants. Examples of suitable crops are listed as follows:
*Allium cepa, Ananas comosus, Arachis hypogaea, Asparagus officinalis, Avena sativa, Beta vulgaris spec.altissima, Beta vulgaris spec.rapa, Brassica napus var.napus, Brassica napus var.napobrassica, Brassica rapavar.silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Hordeum vulgare, Humulus lupulus, Ipomoea batatas, uglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunuspersica, Pyrus communis, Prunus armeniaca, Prunus cerasus, Prunus dulcis and Prunus domestica, Ribes sylvestre, Ricinus communis, Saccharum officinarum, Secale cereale, Sinapisalba, Solanum tuberosum, Sorghum bicolor (S. vulgare), Theobroma cacao, Trifolium pratense, Triticum aestivum, Triticale, Triticum durum, Vicia faba, Vitis vinifera, Zea mays,* rice, or the like.

The compounds I or the herbicidal compositions comprising the compounds I can be used, for example, in the form of ready-to-spray aqueous solutions, powders, suspensions, also highly concentrated aqueous, oily or other suspensions or dispersions, emulsions, oil dispersions, pastes, pulvis, materials for broadcasting, or granules, by means of spraying, atomizing, dusting, spreading, watering or treatment of seeds or mixing with the seeds. The use forms depend on the intended purpose; in each case, they should ensure the finest possible distribution of the active ingredients according to the present disclosure.

The herbicidal composition comprises a herbicidally effective amount of at least one compound of formula I or an agriculturally acceptable salt of I, and auxiliaries commonly used in the formulation of crop protection agents.

Examples of auxiliaries commonly used in the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, colorants (if appropriate) and adhesives for seed formulations.

Examples of thickeners (i.e., compounds which impart to the formulations modified flow properties, i.e., high viscosity in the state of rest and low viscosity in motion state) are polysaccharides, such as xanthan gum, and organic and inorganic sheet minerals.

Examples of antifoams are polysiloxane emulsions, long-chain alcohols, fatty acids, salts of fatty acids, organofluorine compounds, and mixtures thereof.

Examples of antifreeze agents are ethylene glycol, propylene glycol, urea, and glycerin.

Examples of adhesives are polyvinylpyrrolidone, polyvinyl acetate, polyvinyl alcohol, and tylose.

Suitable inert auxiliaries are, for example, the following substances: mineral oil fractions of medium to high boiling point, such as kerosene and diesel oil, furthermore coal tar oils and oils of vegetable or animal origin, aliphatic, cyclic and aromatic hydrocarbons, for example, paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example, amines such as N-methylpyrrolidone, and water.

Solid carriers are mineral earths such as silicas, silica gels, silicates, talc, kaolin, limestone, lime, chalk, bole, loess, clay, dolomite, diatomaceous earth, calcium sulfate, magnesium sulfate and magnesium oxide, grinded synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and emulsifiers) are the alkali metal salts, alkaline earth metal salts and ammonium salts of aromatic sulfonic acids, for example, lignosulfonic acids (e.g. Borrespers-type, Borregaard), phenolsulfonic acids, naphthalenesulfonic acids (Morwet type, Akzo Nobel) and dibutylnaphthalenesulfonic acid (Nekal type, BASF SE), and of fatty acids, alkyl- and alkylaryl-sulfonates, alkyl sulfates, lauryl ether sulfates and fatty alcohol sulfates, and salts of sulfated hexa-, hepta- and octadecanols, and also salts of fatty alcohol glycol ethers, condensates of sulfonated naphthalene and its derivatives with formaldehyde, condensates of naphthalene or the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignin sulfites waste liquors and proteins, denatured proteins, polysaccharides (e.g. methylcellulose), hydrophobically modified starches, polyvinyl alcohol (Mowiol type, Clariant), polycarboxylates (BASF SE, Sokalan type), polyalkoxylates, polyvinylamine (BASF SE, Lupamine type), polyethyleneimine (BASF SE, Lupasol type), polyvinylpyrrolidone and copolymers thereof.

Powders, materials for broadcasting and pulvis can be prepared by mixing or grinding the active ingredients together with solid carriers.

Granules, for example, coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water. To prepare emulsions, pastes or oil dispersions, the compounds of the formula I or la, either as such or dissolved in an oil or solvent, can be homogenized in water by means of a wetting agent, tackifier, dispersant or emulsifier. Alternatively, it is also possible to prepare concentrates comprising active substances, wetting agents, tackifiers, dispersants or emulsifiers and, if desired, solvents or oil, which are suitable for dilution with water.

The concentrations of the compounds of the formula I in the ready-to-use preparations can be varied within wide ranges. In general, the formulations comprise from 0.001 to 98% by weight, preferably 0.01 to 95% by weight of at least one active compound. The active compounds are employed in a purity of from 90% to 100%, preferably 95% to 100% (according to NMR spectrum).

The formulations or the ready-to-use preparations may also comprise acids, bases or buffer systems, suitable examples being phosphoric acid or sulfuric acid, or urea or ammonia.

The compounds I of the present disclosure can for example be formulated as follows.

### 1. Products for Dilution with Water

### A Water-Soluble Concentrates

10 parts by weight of active compound are dissolved in 90 parts by weight of water or a water-soluble solvent. As an alternative, wetting agents or other adjuvants are added. The active compound dissolves upon dilution with water. This gives a formulation with an active compound content of 10% by weight.

### B Dispersible Concentrates

20 parts by weight of active compound are dissolved in 70 parts by weight of cyclohexanone with addition of 10 parts by weight of a dispersant, for example polyvinylpyrrolidone. Dilution with water gives a dispersion. The active compound content is 20% by weight.

### C Emulsifiable Concentrates

15 parts by weight of active compound are dissolved in 75 parts by weight of an organic solvent (e.g., alkylaromatics) with addition of calcium dodecylbenzenesulfonate and castor oil ethoxylate (5 parts by weight in each case). Dilution with water gives an emulsion. The formulation has an active compound content of 15% by weight.

### D Emulsions

25 parts by weight of active compound are dissolved in 35 parts by weight of an organic solvent (e.g., alkylaromatics) with addition of calcium dodecylbenzenesulfonate and castor oil ethoxylate (5 parts by weight in each case). This mixture is introduced into 30 parts by weight of water by means of an emulsifier (e.g., Ultraturrax) and made into a homogeneous emulsion. Dilution with water gives an emulsion. The formulation has an active compound content of 25% by weight.

### E Suspensions

In an agitated ball mill, 20 parts by weight of active compound are comminuted with addition of 10 parts by weight of dispersants and wetting agents and 70 parts by weight of water or an organic solvent, to give a fine active compound suspension. Dilution with water gives a stable suspension of the active compound. The active compound content in the formulation is 20% by weight.

### F Water-Dispersible Granules and Water-Soluble Granules

50 parts by weight of active compound are grinded finely with addition of 50 parts by weight of dispersants and wetting agents, and made into water-dispersible or water-soluble granules by means of industrial unit (for example an extruder, a spray tower, a fluidized bed). Dilution with water gives a stable dispersion or solution of the active compound. The formulation has an active compound content of 50% by weight.

### G Water-Dispersible Powders and Water-Soluble Powders

75 parts by weight of active compound are grinded in a rotor-stator mill with addition of 25 parts by weight of dispersants, wetting agents and silica gel. Dilution with water gives a stable dispersion or solution of the active compound. The formulation has an active compound content of 75% by weight.

### H Gel Formulations

In a ball mill, 20 parts by weight of active compound, 10 parts by weight of dispersant, 1 part by weight of gelling agent and 70 parts by weight of water or of an organic solvent are grinded to give a fine suspension. Dilution with water gives a stable suspension with an active compound content of 20% by weight.

### 2. Undiluted Products to be Applied

### I Pulvis

5 parts by weight of active compound are grinded finely and mixed sufficiently with 95 parts by weight of finely divided kaolin. This gives a dusting powder with an active compound content of 5% by weight.

### J Granules (GR, FG, GG, and MG)

0.5 parts by weight of active compound are grinded finely and associated with 99.5 parts by weight of carriers. Current methods here are extrusion, spray-drying, or the fluidized bed method. This gives undiluted granules to be applied with an active compound content of 0.5% by weight.

### K ULV Solutions (UL)

10 parts by weight of active compound are dissolved in 90 parts by weight of an organic solvent, for example xylene. This gives an undiluted product to be applied with an active compound content of 10% by weight.

The compounds I or the herbicidal compositions comprising them can be applied pre-emergence or post-emergence, or together with seeds of crops. It is also possible to apply the herbicidal compositions or active compounds by applying seeds of crops that are pretreated with the herbicidal compositions or active compounds. If the active compounds are less well tolerated by certain crops, application techniques may be used in which the herbicidal compositions are sprayed, with the aid of the spraying equipment, in such a way that as far as possible they do not come into contact with the leaves of the sensitive crops, while the active ingredients reach the leaves of undesirable plants growing underneath, or the bare soil surface.

In a further embodiment, the compounds of the formula I or the herbicidal compositions comprising the same can be applied by treating seeds.

The treatment for seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of the formula I according to the present disclosure or the compositions prepared therefrom. Here, the diluted or undiluted herbicidal compositions can be applied.

The term "seed" comprises seed of all types, such as, for example, grains, seeds, fruits, tubers, cuttings, and similar forms. Here, preferably, the term "seed" describes grains and seeds.

The seed used can be seed of the useful plants mentioned above, but also the seed of transgenic plants or plants obtained by customary breeding methods.

The active compound is applied at a rate of from 0.001 to 3.0 kg/ha, preferably 0.01 to 1.0 kg/ha, of active substance (a.s.), depending on the control target, the season, the target plants and the growth stage. To treat the seed, the compounds I are generally employed in amounts from 0.001 to 10 kg per 100 kg of seed. It may also be advantageous to use the compounds of the formula I in combination with safeners. Safeners are compounds which prevent or reduce damage to useful plants without substantially affecting the herbicidal action of the compounds of the formula I on unwanted plants. They can be used both before sowing (for example in the treatment of seed, or on cuttings or seedlings) and before or after the emergence of the useful plant. The safeners and the compounds of the formula I can be used simultaneously or in succession. Suitable safeners are, for example, (quinolin-8-oxy)acetic acids, 1-phenyl-5-haloalkyl-1H-1,2,4-triazole-3-carboxylic acids, 1-phenyl-4,5-dihydro-5-alkyl-1H-pyrazole-3,5-dicarboxylic acids, 4,5-dihydro-5,5-diaryl-3-isoxazole carboxylic acids, dichloroacetamides, α-oximinophenylacetonitriles, acetophenone oximes, 4,6-dihalo-2-phenylpyrim idines, N-[[4-(aminocarbonyl)phenyl]sulfonyl]-2-benzamides, 1,8-naphthalicanhydride, 2-halo-4-haloalkyl-5-thiazole carboxylic acids, phosphorothioates and O-phenyl N-alkylcarbamates and their agriculturally acceptable salts, and their agriculturally acceptable derivatives, such as amides, esters and thioesters, provided that they have acidic functional groups.

To broaden the activity spectrum and to obtain synergistic effects, the compounds of the formula I can be mixed and jointly applied with numerous representatives of other herbicidal or growth-regulating active compounds, or with safeners. Suitable mixing partners are, for example, 1,2,4-thiadiazoles, 1,3,4-thiadiazoles, amides, aminophosphoric acid and its derivatives, aminotriazoles, anilides, aryloxy/heteroaryloxyalkanoic acids and their derivatives, benzoic acid and its derivatives, benzothiadiazine ketones, 2-hetaroyl/aroyl-1,3-cyclohexanediones, heteroaryl aryl ketones, benzylisoxazole alkyl ketones, meta-CF₃-phenyl derivatives, carbamates, quinoline carboxylic acid and its derivatives, chloroacetanilides, cyclohexenone oxime ether derivates, diazines, dichloropropionic acid and its derivatives, dihydrobenzofurans, dihydrofuran-3-ones, dinitroanilines, dinitrophenols, diphenyl ethers, dipyridyls, halocarboxylic acids and their derivatives, ureas, 3-phenyluracils, imidazoles, imidazolinones, N-phenyl-3,4,5,6-tetrahydrophthalimides, oxadiazoles, oxiranes, phenols, aryloxy- and heteroaryloxyphenoxypropionic esters, phenylacetic acid and its derivatives, 2-phenylpropionic acid and its derivatives, pyrazoles, phenylpyrazoles, pyridazines, pyridinecarboxylic acid and its derivatives, pyrimidyl ethers, sulfonamides, sulfonylureas, triazines, triazinones, triazolinones, triazolecarboxamides, uracils, phenylpyrazolines, and isoxazolines and their derivatives.

Moreover, it may be useful to apply the compounds I alone or in combination with other herbicides or else also mixed with other crop protection agents, jointly, for example with compositions for controlling pests or phytopathogenic fungi or bacteria. Also of interest is the miscibility with mineral salt solutions which are employed for alleviating nutritional and trace element deficiencies. Other additives such as nonphytotoxic oils and oil concentrates may also be added.

Examples of herbicides which can be used in combination with the pyridine compounds of the formula I according to the present invention are:
b1) selected from the following lipid biosynthesis inhibitors: clethodim, clodinafop, clodinafop-propargyl, cycloxydim, cyhalofop, cyhalofop-butyl, diclofop, diclofop-methyl, fenoxaprop-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) selected from the following ALS inhibitors: bensulfuron, bensulfuron-methyl, bispyribac, cinosulfuron, cloransulam, cloransulam-methyl, cyclosulfamuron, diclosulam, ethametsulfuron, ethametsulfuron-methyl, ethoxysulfuron, flazasulfuron, florasulam, flucarbazone, flucetosulfuron, flumetsulam, flupyrsulfuron, flupyrsulfuron-methyl-sodium, foramsulfuron, halosulfuron, halosulfuron-methyl, imazosulfuron, iodosulfuron, iodosulfuron-methyl-sodium, mesosulfuron, metosulam, metsulfuron, metsulfuron-methyl, nicosulfuron, orthosulfamuron, oxasulfuron, penoxsulam, primisulfuron, primisulfuron-methyl, propoxycarbazone, propoxycarbazone-sodium, prosulfuron, pyrazosulfuron, pyrazosulfuron-ethyl, pyribenzoxim, pyrimisulfan, pyriftalid, pyriminobac, pyriminobac-methyl, pyroxsulam, thifensulfuron, thifensulfuron-methyl, triasulfuron, tribenuron, tribenuron-methyl, trifloxysulfuron, triflusulfuron, triflusulfuron-methyl and tritosulfuron; imazamethabenz, imazamethabenz-methyl, imazamox, imazapic, imazapyr, imazaquin, imazethapyr, imazosulfuron, iodosulfuron, iodosulfuron-methyl-sodium, mesosulfuron, metosulam, metsulfuron, metsulfuron-methyl, nicosulfuron, orthosulfamuron, oxasulfuron, penoxsulam, primisulfuron, primisulfuron-methyl, propoxycarbazone, propoxycarbazone-sodium, prosulfuron, pyrazosulfuron, pyrazosulfuron-ethyl and pyribenzoxim;
b3) selected from the following photosynthesis inhibitors: ametryn, amicarbazone, atrazine, bentazone, bentazone-sodium, bromacil, bromofenoxim, bromoxynil and its salts and esters, chlorobromuron, chloridazone, cyanazine, desmedipham, desmetryn, diquat-dibromide, diuron, fluometuron, hexazinone, ioxynil and its salts and esters, isoproturon, isouron, karbutilate, lenacil, linuron, metamitron, methabenzthiazuron, paraquat-dimetilsulfate, pentanochlor, phenmedipham, phenmedipham-ethyl, prometon, prometryn, propanil, propazine, pyridafol, pyridate, siduron, simazine, simetryn, terbacil and terbuthylazine;
b4) selected from the following protoporphyrinogen-IX oxidase inhibitors: acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, 2-chloro-5-[3,6-dihydro-3-methyl-2,6-dioxo-4-trifluoromethyl-[(2H)-pyrimidinyl]-4-fluoro-N-[(isopropyl)-methylsulfamoyl]benzamide (H-1; CAS 372137-35-4), ethyl
   [3-[2-chloro-4-fluoro-5-(1-methyl-6-trifluoromethyl-2,4-dioxo-1,2,3,4-tetrahydro pyrimidin-3-yl)phenoxy]-2-pyridyloxy]acetate (H-2; CAS 353292-31-6), N-ethyl-3-(2,6-dichloro-4-trifluoromethylphenoxy)-5-methyl-1H-pyrazole-1-carb oxamide (H-3; CAS 452098-92-9), N-tetrahydrofurfuryl-3-(2,6-dichloro-4-trifluoromethylphenoxy)-5-methyl-1H-pyr azole-1-carboxamide (H-4; CAS 915396-43-9), N-ethyl-3-(2-chloro-6-fluoro-4-trifluoromethylphenoxy)-5-methyl-1H-pyrazole-1 -carboxamide (H-5; CAS 452099-05-7), N-tetrahydrofurfuryl-3-(2-chloro-6-fluoro-4-trifluoromethylphenoxy)-5-methyl-1 H-pyrazole-1-carboxamide (H-6; CAS 45100-03-7); 3-[7-fluoro-3-oxo-4-(prop-2-ynyl)-3,4-dihydro-2H-benzo[1,4]oxazine-6-yl]-1,5-d imethyl-6-thio-[1,3,5]triazinan-2,4-dione,
   1,5-dimethyl-6-thio-3-(2,2,7-trifluoro-3-oxo-4-(prop-2-ynyl)-3,4-dihydro-2H-ben zo[b][1,4]oxazine-6-yl)-1,3,5-triazinan-2,4-dione and 2-(2,2,7-trifluoro-3-oxo-4-prop-2-ynyl-3,4-dihydro-2H-benzo[1,4]oxazine-6-yl)-4,5,6,7-tetrahydroisoind ole-1,3-dione and 1-methyl-6-trifluoromethyl-3-(2,2,7-trifluoro-3-oxo-4-prop-2-ynyl-3,4-dihydro-2 H-benzo[1,4]oxazine-6-yl)-1H-pyrimidine-2,4-dione;
   a compound represented by the following formula 2: 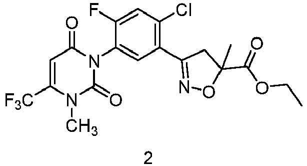
b5) selected from the following bleacher herbicides: aclonifen, amitrol, beflubutamid, benzobicyclon, benzofenap, clomazone, diflufenican, fluridone, flurochloridone, flurtamone, isoxaflutole, mesotrione, norflurazon, picolinafen, pyrasulfutole, pyrazolynate, pyrazoxyfen, sulcotrione, tefuryltrione, tembotrione, topramezone, bixlozone, tripyrasulfone, cypyrafluone, bipyrasulfone,
   4-hydroxy-3-[[2-[(2-methoxyethoxy)methyl]-6-(trifluoromethyl)-3-pyridyl]carbon yl]bicyclo[3.2.1]oct-3-en-2-one (H-7; CAS 352010-68-5) and 4-(3-trifluoromethylphenoxy)-2-(4-trifluoromethylphenyl)pyrimidine (H-8; CAS 180608-33-7);
b6) selected from the following EPSP synthase inhibitors: glyphosate, glyphosate-isopropylammonium and glyphosate-trimesium (sulfosate);
b7) selected from the following glutamine synthase inhibitors: bilanaphos (bialaphos), bilanaphos-sodium, and glufosinate-ammonium;
b8) selected from the following DHP synthase inhibitors: asulam;
b9) selected from the following mitose inhibitors: amiprophos, amiprophos-methyl, benfluralin, butamiphos, butralin, carbetamide, chlorpropham, chlorthal, chlorthal-dimethyl, dinitramine, dithiopyr, ethalfluralin, fluchloralin, oryzalin, pendimethalin, prodiamine, propham, propyzamide, tebutam, thiazopyr and trifluralin;
b10) selected from the following VLCFA inhibitors: acetochlor, alachlor, anilofos, butachlor, cafenstrole, dimethachlor, dimethanamid, dimethenamid-P, diphenamid, fentrazamide, flufenacet, mefenacet, metazachlor, metolachlor, metolachlor-S, naproanilide, napropamide, pethoxamid, piperophos, pretilachlor, propachlor, propisochlor, pyroxasulfone (KIH-485) and thenylchlor;
b11) selected from the following cellulose biosynthesis inhibitors: chlorthiamid, dichlobenil, flupoxam and isoxaben;
b12) selected from the following separating agent herbicides: dinoseb, dinoterb, and DNOC and its salts;
b13) selected from the following auxin herbicides: 2,4-D and its salts and esters, 2,4-DB and its salts and esters, aminopyralid and its salts such as aminopyralid-tris(2-hydroxypropyl)ammonium and its esters, benazolin, benazolin-ethyl, chioramben and its salts and esters, clomeprop, clopyralid and its salts and esters, dicamba and its salts and esters, dichlorprop and its salts and esters, dichlorprop-P and its salts and esters, fluoroxypyr, fluoroxypyr-butomethyl, fluoroxypyr-meptyl, MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, and 5,6-dichloro-2-cyclopropyl-4-pyrimidineformic acid (H-9; CAS 858956-08-8) and its salts and esters;
b14) selected from the following auxin transport inhibitors: diflufenzopyr and naptalam;
b15) selected from the following other herbicides: bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, flamprop, flamprop-M-isopropyl, flamprop-M-methyl, flurenol, flurenol-butyl, flurprimidol, fosamine, fosamine-ammonium, indanofan, maleic hydrazide, mefluidide, methyl-dymron, oxaziclomefone, pelargonic acid, pyributicarb, and 6-chloro-3-(2-cyclopropyl-6-methylphenoxy)-4-pyridazinol (H-10; CAS 499223-49-3) and its salts and esters.

Examples of preferred safeners C are benoxacor, cloquintocet, cyometrinil, cyprosulfamide, dichlormid, dicyclonone, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (H-11; MON4660, CAS 71526-07-3), and 2,2,5-trimethyl-3-(dichloroacetyl)-1,3-oxazolidine (H-12; R-29148, CAS 52836-31-4).

The active compounds of groups b1) to b15) and the safeners are known herbicides and safeners, see, for example, The Compendium of Pesticide Common Names (http://www.alanwood.net/pesticides/); B. Hock, C. Fedtke, R. R. Schmidt, Herbizide, Georg Thieme Verlag, Stuttgart, 1995. Further herbicidally active compounds are known from WO 96/26202, WO 97/41116, WO 97/41117, WO 97/41118, WO 01/83459 and WO 2008/074991 and from W. Kramer et al. (ed.) "Modern Crop Protection Compounds", Vol. 1, Wiley VCH, 2007 and the literature quoted therein.

The present disclosure also relates to compositions in the form of a crop protection composition formulated as a single-component composition comprising an active compound combination containing at least one pyridazine compound of the formula I and at least one further active compound, preferably selected from the active compounds of groups b1 to b15, and at least one solid or liquid carrier and/or one or more surfactants and, if desired, one or more further auxiliaries customary for crop protection compositions.

The present disclosure also relates to compositions in the form of a crop protection composition formulated as a dual-component composition comprising a first component containing at least one pyridazine compound of the formula I, a solid or liquid carrier and/or one or more surfactants, and a second component containing at least one further active compound selected from the active compounds of groups b1 to b15, a solid or liquid carrier and/or one or more surfactants, wherein both components may also additionally comprise further auxiliaries customary for crop protection compositions.

The compounds I and the compositions according to the present disclosure may also have a plant-strengthening action. Accordingly, they are suitable for mobilizing the defense system of the plants against attack by unwanted microorganisms, such as harmful fungi, but also viruses and bacteria. Plant-strengthening (resistance-inducing) substances are to be understood as meaning, in the present context, those substances which are capable of stimulating the defense system of treated plants in such a way that, when subsequently inoculated by unwanted microorganisms, the treated plants display a substantial degree of resistance to these microorganisms.

The compounds I can be employed for protecting plants against attack by unwanted microorganisms within a certain period of time after the treatment. The period of time within which their protection is effected generally extends from 1 to 28 days, preferably from 1 to 14 days, after the treatment of the plants or after the treatment of the seed with the compounds I, for up to 9 months after sowing.

The compounds I and the compositions according to the present disclosure are also suitable for increasing the harvest yield.

Hereinbelow, the compounds of the formula I are illustrated by way of examples, without limiting the subject matter of the present disclosure to the examples shown.

### Detailed Description of the Embodiments

### Synthesis Examples

### Example 1: Preparation of Compound I-A-8

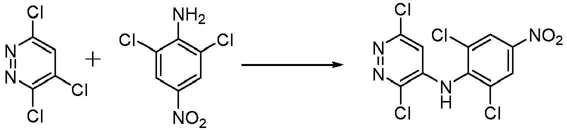

18.3 grams of 3,4,6-trichloropyridazine, 20.7 grams of 2,6-dichloro-4-nitroaniline, 15 grams of anhydrous potassium carbonate, and 200 ml of anhydrous acetonitrile were put into a 500 ml four-neck flask while being stirred at room temperature. The mixture was heated to a temperature of 60 °C and was reacted while being stirred for 3 hours. After the reaction was completed, the solvent was removed and the residue was extracted with 300 ml of ethyl acetate. 20.1 grams of a product was obtained by column chromatography with a melting point of 207 to 209 °C. δppm: 6.35 (1H, s), 8.18-8.23 (2H, s), 8.40-8.43 (1H, s).

### Example 2: Preparation of Compound I-B-1

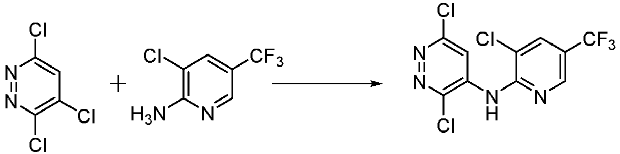

18.3 grams of 3,4,6-trichloropyridazine, 19.6 grams of 3-chloro-5-trifluoromethyl-2-pyridinamine, 18 grams of anhydrous potassium carbonate, and 200 ml of anhydrous acetonitrile were put into a 500 ml four-neck flask while being stirred at room temperature. The mixture was heated to a temperature of 60 °C and was reacted while being stirred for 3 hours. After the reaction was completed, the solvent was removed and the residue was extracted with 300 ml of ethyl acetate. 18.3 grams of a product was obtained by column chromatography with a melting point of 140 to 142 °C. δppm: 7.95-8.05 (1H, s), 8.30-8.40 (1H, s), 8.60-8.70 (1H, s), 9.00-9.10 (1H, s).

### Example 3: Preparation of Compound I-I-1

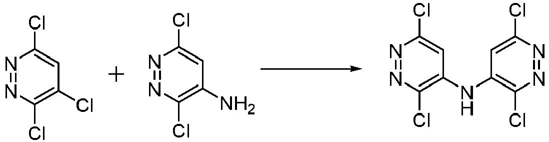

36.6 grams of 3,4,6-trichloropyridazine, 33 grams of 3,6-dichloro-4-aminopyridazine, 35 grams of anhydrous potassium carbonate, and 300 ml of anhydrous acetonitrile were put into a 500 ml four-neck flask while being stirred at room temperature. The mixture was heated to a temperature of 60 °C and was reacted while being stirred for 3 hours. After the reaction was completed, the solvent was removed and the residue was extracted with 500 ml of ethyl acetate. 30.6 grams of a product was obtained by column chromatography with a melting point of 179 to 181 °C. δppm: 7.45-7.48 (2H, s).

### Example 4: Preparation of Compound I-O-2

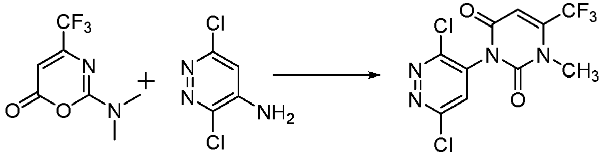

36.6 grams of 3,4,6-trichloropyridazine and 41.6 grams of 2-dimethylamino-4-trifluoromethyl-6H-1,3-oxazine-6-one were added to a reaction flask containing 200 ml of acetic acid while being stirred at room temperature. The mixture was heated to a reflux reaction to form a dark solution. The reaction was maintained at this temperature for 6 hours. Then, the solvent was removed under reduced pressure. The residue was adjusted to a neutral pH with addition of an aqueous solution of sodium bicarbonate and was extracted with ethyl acetate, and the solvent was removed to give a crude product. 38.5 grams of a product was obtained by column chromatography.

Other compounds of general formula (I) could be prepared by the preparation methods according to the present disclosure.

The melting points (measured by an uncalibrated melting point apparatus) and NMR data (¹HNMR, 400 MHz, internal standard: TMS, solvent: CDCl₃) of some compounds were shown as follows:
Compound I-A-8: having a melting point of 207 to 209 °C. δppm: 6.35 (1H, s), 8.18-8.23 (2H, s), 8.40-8.43 (1H, s).
Compound I-B-1: having a melting point of 140 to 142 °C. δppm: 7.95-8.05 (1H, s), 8.30-8.40 (1H, s), 8.60-8.70 (1H, s), 9.00-9.10 (1H, s).
Compound I-A-17: having a melting point of 149 to 151 °C. δppm: 6.60-6.66 (1H, s), 7.12-7.18 (1H, s).
Compound I-A-19: δppm 6.70-6.74 (1H, s), 8.70-8.72 (1H, s).
Compound I-I-1: having a melting point of 179 to 181 °C. δppm: 7.45-7.48 (2H, s).
Compound I-I-7: having a melting point of 143 to 147 °C. δppm: 2.96-2.98 (3H, d), 7.26 (2H, s).

### II. Use Examples

The herbicidal activity of the compounds of the formula I was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This covering caused uniform germination of the test plants, unless this has been impaired by the active compounds.

For the post-emergence treatment, the test plants were first grown to a height of 3 to 15 cm, depending on the plant habit, and then, only at this time, treated with the active ingredients which had been suspended or emulsified in water. For this purpose, the test plants were either sown directly or grown in the same containers, or they were first grown separately as seedlings and transplanted into the test containers a few days prior to treatment.

Depending on the species, the plants were kept at 10 to 25 °C or at 20 to 35 °C. The test period extended over 2 to 4 weeks. During this time, the plants were tended, and their response to the individual treatments was evaluated.

Evaluation was carried out using a score from 0 to 100. 100 means no emergence of the plants, or complete destruction of at least the part on the ground, and 0 means no damage, or normal course of growth. A good herbicidal activity is given at a score of at least 70 and a very good herbicidal activity is given at a score of at least 85.

The plants used in the greenhouse experiments belonged to the following species:

| Bayer Code | Scientific Name | Common Name |
|---|---|---|
| AMARE | *Amaranthus retroflexus* | Redroot pigweed |
| CHEAL | *Chenopodium album* | Lambsquarters |
| ECHCG | *Echinochloa crus-galli* | Barnyardgrass |
| GALAP | *Galium aparine* | Catchweed bedstraw |
| ALOMY | *Alopercurus myosuroides* | Amur foxtail |
| ELEIN | *Eleusine indica* | Goosegrass |
| POAAN | *Poa annua* | Annual bluegrass |

At an application rate of 0.5 kg/ha, the compounds I-A-8, I-I-1, and I-A-19 applied by the post-emergence method showed very good herbicidal activity against *Eleusine indica* (Goosegrass).

At an application rate of 0.5 kg/ha, the compound I-I-1 applied by the post-emergence method showed very good herbicidal activity against *Echinochloa crus-galli* (Barnyardgrass).

At an application rate of 0.5 kg/ha, the compound I-I-1 applied by the post-emergence method showed very good herbicidal activity against *Alopercurus myosuroides* (Amur foxtail).

At an application rate of 0.5 kg/ha, the compound I-I-1 applied by the post-emergence method showed very good herbicidal activity against *Poa annua* (Annual bluegrass).

## Claims

1. A pyridazinyl amine compound represented by general formula (I), or an N-oxide or agriculturally acceptable salt of the compound of the general formula (I), 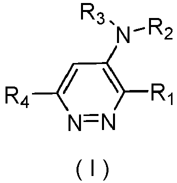
**characterized in that**
R₁ is selected from hydrogen, halogen, cyano, and phenoxy or heteroaryloxy unsubstituted or substituted with one to four groups independently selected from the group consisting of: halogen, nitro, cyano, C₁-C₁₂ alkyl, halogenated C₁-C₁₂ alkyl, C₁-C₁₂ alkoxy, halogenated C₁-C₁₂ alkoxy, C₃-C₁₂ cycloalkyl, C₁-C₁₂ alkylcarbonyl and C₁-C₁₂ alkoxycarbonyl;
R₂ is selected from hydrogen, C₁-C₁₂ alkyl, halogenated C₁-C₁₂ alkyl, C₁-C₁₂ alkoxy, halogenated C₁-C₁₂ alkoxy, C₃-C₆ cycloalkyl, cyano C₁-C₁₂ alkyl, C₁-C₁₂ alkylamino, halogenated C₁-C₁₂ alkylamino, C₂-C₆ dialkylamino, C₂-C₁₂ alkenyl, halogenated C₂-C₁₂ alkynyl, C₂-C₁₂ alkenyloxy, halogenated C₂-C₁₂ alkenyloxy, C₂-C₁₂ alkynyl, halogenated C₂-C₁₂ alkenyl, C₂-C₁₂ alkynyloxy, halogenated C₂-C₁₂ alkynyloxy, C₁-C₁₂ alkylsulfonyl, C₁-C₁₂ alkylcarbonyl, halogenated C₁-C₁₂ alkylcarbonyl, or halogenated C₁-C₁₂ alkylsulfonyl;
R₃ is selected from phenyl, benzyl or heteroaryl unsubstituted or substituted with one to four groups independently selected from the group consisting of: halogen, hydroxyl, nitro, cyano, CHO, COOH, COONa, C₁-C₁₂ alkyl, halogenated C₁-C₁₂ alkyl, C₁-C₁₂ alkoxy, halogenated C₁-C₁₂ alkoxy, C₃-C₆ cycloalkyl, cyano C₁-C₁₂ alkyl, C₁-C₁₂ alkylamino, halogenated C₁-C₁₂ alkylamino, C₂-C₆ dialkylamino, C₂-C₁₂ alkenyl, halogenated C₂-C₁₂ alkynyl, C₂-C₁₂ alkenyloxy, halogenated C₂-C₁₂ alkenyloxy, C₂-C₁₂ alkynyl, halogenated C₂-C₁₂ alkenyl, C₂-C₁₂ alkynyloxy, halogenated C₂-C₁₂ alkynyloxy, C₁-C₁₂ alkylthio, halogenated C₁-C₁₂ alkylthio, C₁-C₁₂ alkylsulfonyl, C₁-C₁₂ alkylcarbonyl, halogenated C₁-C₁₂ alkylcarbonyl and halogenated C₁-C₁₂ alkylsulfonyl;
R₂NR₃ is able to form a five-membered or six-membered ring unsubstituted or substituted with one to four groups independently selected from the group consisting of: halogen, hydroxyl, nitro, cyano, CHO, COOH, COONa, C₁-C₁₂ alkyl, halogenated C₁-C₁₂ alkyl, C₃-C₁₂ cycloalkyl, C₁-C₁₂ alkoxy, halogenated C₁-C₁₂ alkoxy, C₁-C₁₂ alkylthio, C₁-C₁₂ alkylcarbonyl and C₁-C₁₂ alkoxycarbonyl; and
R₄ is selected from halogen, cyano, CHO, COOH, COONa, C₁-C₁₂ alkylsulfonyl, halogenated C₁-C₁₂ alkylsulfonyl, or arylsulfonyl unsubstituted or substituted with one to four groups independently selected from the group consisting of: halogen, hydroxyl, nitro, cyano, CHO, COOH, COONa, C₁-C₁₂ alkyl, halogenated C₁-C₁₂ alkyl, C₃-C₁₂ cycloalkyl, C₁-C₁₂ alkoxy, halogenated C₁-C₁₂ alkoxy, C₁-C₁₂ alkylthio, C₁-C₁₂ alkylcarbonyl and C₁-C₁₂ alkoxycarbonyl.

2. The pyridazinyl amine compound or an N-oxide or agriculturally acceptable salt of the compound of the general formula (I) according to claim 1, wherein in the general formula (I),
R₁ is selected from hydrogen, Cl, Br, F, I, cyano, and phenoxy or heteroaryloxy unsubstituted or substituted with one to four groups independently selected from the group consisting of: halogen, nitro, cyano, C₁-C₆ alkyl, halogenated C₁-C₆ alkyl, C₁-C₆ alkoxy, halogenated C₁-C₆ alkoxy, C₃-C₆ cycloalkyl, C₁-C₆ alkylcarbonyl or C₁-C₆ alkoxycarbonyl;
R₂ is selected from hydrogen, C₁-C₆ alkyl, halogenated C₁-C₆ alkyl, C₁-C₆ alkoxy, halogenated C₁-C₆ alkoxy, C₃-C₆ cycloalkyl, cyano C₁-C₆ alkyl, C₁-C₆ alkylamino, halogenated C₁-C₆ alkylamino, C₂-C₆ dialkylamino, C₂-C₆ alkenyl, halogenated C₂-C₆ alkenyl, C₂-C₆ alkenyloxy, halogenated C₂-C₆ alkenyloxy, C₂-C₆ alkynyl, halogenated C₂-C₆ alkynyl, C₂-C₆ alkynyloxy, halogenated C₂-C₆ alkynyloxy, C₁-C₆ alkylsulfonyl, C₁-C₆ alkylcarbonyl, halogenated C₁-C₆ alkylcarbonyl, or halogenated C₁-C₆ alkylsulfonyl;
R₃ is selected from phenyl, benzyl or heteroaryl unsubstituted or substituted with one to four groups independently selected from the group consisting of: Cl, Br, F, I, hydroxyl, nitro, cyano, CHO, COOH, COONa, C₁-C₆ alkyl, halogenated C₁-C₆ alkyl, C₁-C₆ alkoxy, halogenated C₁-C₆ alkoxy, C₃-C₆ cycloalkyl, cyano C₁-C₆ alkyl, C₁-C₆ alkylamino, halogenated C₁-C₆ alkylamino, C₂-C₆ dialkylamino, C₂-C₆ alkenyl, halogenated C₂-C₆ alkynyl, C₂-C₆ alkenyloxy, halogenated C₂-C₆ alkenyloxy, C₂-C₆ alkynyl, halogenated C₂-C₆ alkenyl, C₂-C₆ alkynyloxy, halogenated C₂-C₆ alkynyloxy, C₁-C₆ alkylthio, halogenated C₁-C₆ alkylthio, C₁-C₆ alkylsulfonyl, C₁-C₆ alkylcarbonyl, halogenated C₁-C₆ alkylcarbonyl and halogenated C₁-C₆ alkylsulfonyl;
R₂NR₃ is able to form a five-membered or six-membered ring unsubstituted or substituted with one to four groups independently selected from the group consisting of: Cl, Br, F, I, hydroxyl, nitro, cyano, CHO, COOH, COONa, C₁-C₆ alkyl, halogenated C₁-C₆ alkyl, C₃-C₆ cycloalkyl, C₁-C₆ alkoxy, halogenated C₁-C₆ alkoxy, C₁-C₆ alkylthio, C₁-C₆ alkylcarbonyl and C₁-C₆ alkoxycarbonyl; and
R₄ is selected from Cl, Br, F, I, cyano, CHO, COOH, COONa, C₁-C₆ alkylsulfonyl, or halogenated C₁-C₆ alkylsulfonyl.

3. The pyridazinyl amine compound or an N-oxide or agriculturally acceptable salt of the compound of the general formula (I) according to claim 2, wherein in the general formula (I),
R₁ is selected from H, Cl, Br, F, I, CN, CH₃, CH₂CH₃, tert-butyl, cyclopropyl, CF₃, CH₂CF₃, OCH₃, OCF₃, OCH₂CF₃, SO₂CH₃, CO₂CH₃, and phenoxy unsubstituted or substituted with a substituent(s) selected from one to three of Cl, Br, F, I, CN, NO₂, CHO, COOH, COONa, CH₃, CH₂CH₃, tert-butyl, cyclopropyl, CHF₂, CF(CF₃)₂, CF₃, CH₂CF₃, OCH₃, OCHF₂, OCF₃, OCH₂CF₃ and SO₂CH₃;
R₂ is selected from H, methyl, ethyl, propyl, isopropyl, tert-butyl, n-butyl, methoxy, cyclopropyl, cyanoethyl, methylamino, methanesulfonyl, allyl, propargyl, methanesulfonyl, p-toluenesulfonyl, ethylcarbonyl, or formyl;
R₃ is selected from phenyl, benzyl or heteroaryl unsubstituted or substituted with one to three groups independently selected from the group consisting of: OH, Cl, Br, F, I, CN, NO₂, CHO, COOH, COONa, CH₃, CH₂CH₃, tert-butyl, cyclopropyl, CHF₂, CF₃, CF(CF₃)₂, CH₂CF₃, OCH₃, OCHF₂, OCF₃, OCH₂CF₃ and SO₂CH₃;
R₂NR₃ is able to form a five-membered or six-membered ring unsubstituted or substituted with one to three groups independently selected from the group consisting of: OH, Cl, Br, F, I, CN, NO₂, CH₃, CH₂CH₃, tert-butyl, cyclopropyl, CHF₂, CF(CF₃)₂, CF₃, CH₂CF₃, OCH₃, OCHF₂, OCF₃, OCH₂CF₃ and SO₂CH₃; and
R4 is selected from Cl, Br, F, I, CN, SO₂CH₃, or SO₂CH₂CH₃; and
the salt is formed by the compound of the general formal (I) and hydrochloric acid, sulfuric acid, phosphoric acid, formic acid, acetic acid, oxalic acid, trifluoroacetic acid, methanesulfonic acid, p-toluenesulfonic acid, malic acid, citric acid, lithium, calcium, sodium, or potassium.

4. The pyridazinyl amine compound or an N-oxide or agriculturally acceptable salt of the compound of the general formula (I) according to claim 3, wherein in the general formula (I),
R₁ is selected from Cl, Br, F, I, 2-methylphenol, 2-isopropylphenol, 2-cyclopropylphenol, 2-methyl-6-cyclopropylphenol, or 2-iodophenol;
R₂ is selected from H, CH₃, CH₂CH₃, cyclopropyl, methoxy, or methylamino;
R₃ is selected from phenyl, pyrimidinyl, pyrazolyl, pyridinyl or pyridazinyl unsubstituted or substituted with one to three groups independently selected from the group consisting of: OH, Cl, Br, F, I, CN, NO₂, CHO, COOH, COONa, CH₃, CH₂CH₃, tert-butyl, cyclopropyl, CHF₂, CF₃, CH₂CF₃, OCH₃, OCHF₂, OCF₃, CF(CF₃)₂, OCH₂CF₃ and SO₂CH₃;
R₂NR₃ is able to form morpholine, piperazine, piperidine, a pyrrole ring, a pyrazole ring, a triazole ring, a pyrimidine ring, or 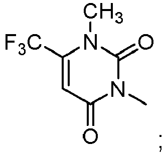 and
R₄ is selected from Cl, Br, F, I, or CN; and
the salt is formed by the compound of the general formula (I) and hydrochloric acid, sulfuric acid, phosphoric acid, lithium, calcium, sodium, or potassium.

5. The pyridazinyl amine compound or an N-oxide or agriculturally acceptable salt of the compound of the general formula (I) according to claim 4, wherein in the general formula (I),
R₁ is selected from Cl, Br, F, or I;
R₂ is selected from H, CH₃, or CH₂CH₃;
R₃ is selected from phenyl, pyridyl or pyridazinyl unsubstituted or substituted with one to three groups independently selected from the group consisting of: OH, Cl, Br, F, I, CN, NO₂, CHO, COOH, COONa, CH₃, CH₂CH₃, tert-butyl, CHF₂, CF₃, CH₂CF₃, CF(CF₃)₂, OCH₃, OCHF₂, OCF₃, OCH₂CF₃ or SO₂CH₃;
R₂NR₃ is able to form a ring 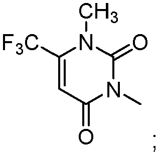 and
R₄ is selected from Cl, Br, F, I, or CN; and
the salt is formed by the compound of the general formal (I) and hydrochloric acid, sulfuric acid, phosphoric acid, lithium, calcium, sodium, or potassium.

6. Use of the pyridazinyl amine compound of general formula (I) according to claim 1 in pre-emergence treatment or post-emergence treatment of stems and leaves for control of weeds.

7. Use of the pyridazinyl amine compound of general formula (I) according to claim 1 for desiccation/defoliation of a plant.

8. A herbicidal composition, **characterized by** comprising the pyridazinyl amine compound of general formula (I) according to claim 1 as an active component, wherein the composition comprises, by weight percentage, 0.1 to 99% of the active component.

9. Use of the herbicidal composition according to claim 8 for control of weeds.

10. The herbicide composition according to claim 8, wherein the herbicide composition is able to be applied in combination with other known insecticides, microbicide, plant growth regulators or fertilizers, including herbicide and safener.
